(19) Europäisches Patentamt
European Patent Office
Office européen des brevets

(11) Veröffentlichungsnummer: **0 363 856 A1**

# (12) EUROPÄISCHE PATENTANMELDUNG

(21) Anmeldenummer: **89118663.7**

(51) Int. Cl.5: **C07D 311/16 , C12Q 1/48**

(22) Anmeldetag: **07.10.89**

Patentansprüche für folgenden Vertragsstaat: ES.

(30) Priorität: **11.10.88 DE 3834597**

(43) Veröffentlichungstag der Anmeldung: **18.04.90 Patentblatt 90/16**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Aretz, Werner, Dr.**
**Am Wäldchen 13**
**D-6240 Königstein/Taunus(DE)**
Erfinder: **Hedtmann, Udo, Dr.**
**Liederbacher Strasse 6**
**D-6230 Frankfurt am Main 80(DE)**

## (54) Neues Cumarinderivat, Verfahren zu seiner Herstellung und seine Verwendung.

(57) Mit 7-(δ-(D-α-Aminoadipinyl)-amido)-4-methylcumarin wurde ein Ersatz für Adipinyl-p-nitroanilid als Reagens für γ-Glutamyl-Transpeptidasen mit α-Adipinylamidase Aktivität gefunden. Bei der Hydrolyse des neuen Reagenz wird stark fluoreszierendes 7-Amino-4-methylcumarin freigesetzt, das leicht nachgewiesen werden kann.

EP 0 363 856 A1

## Neues Cumarinderivat, Verfahren zu seiner Herstellung und seine Verwendung

Die in der Therapie angewandten β-Lactam-Antibiotika aus der Gruppe der Cephalosporine sind ausschließlich semisynthetischer Natur und enthalten als Nucleus die 7-Aminocephalosporansäure (7ACA), gewonnen aus Cephalosporin C (CPC). Die Spaltung von CPC erfolgt entweder chemisch oder enzymatisch. Für letztere braucht man entweder die Enzymkombination D-Aminosäure-Oxidase/γ-Glutamyl-Transpeptidase (EP 275 901) oder eine CPC-Amidase-Aktivität (DE 37 43 323).

Für das Auffinden von Cephalosporin C (CPC)-Amidase produzierenden Mikroorganismen wurde von R.B. Walton [Developments in Industrial Microbiology, 5, 341-353, (1964)] ein Screening-Verfahren beschrieben, bei dem N-Adipinyl-p-nitroanilid als Substrat verwendet und das entstandene p-Nitroanilin aufgrund seiner Gelbfärbung bestimmt wurde. Nachteil dieses Verfahrens ist die geringe Spezifität des Substrats und die Überlagerung der Gelbfärbung durch Bestandteile von Nährlösungen.

Es wurde überraschend gefunden, daß die Überlagerung der Gelbfärbung durch Nährmedienbestandteile durch ein neues Substrat, 7-(δ-(D-α-Aminoadipinyl)-amido)-4-methylcumarin, das nach der Hydrolyse die stark fluoreszierende Substanz 7-Amino-4-methylcumarin freisetzt, umgangen werden kann.

γ-Glutamyl-Transpeptidasen (im folgenden γ-GTP) spielen in tierischen Geweben und in Mikroorganismen eine wichtige Rolle im Aminosäurestoffwechsel und im Glutathion-Zyklus [Meth. Enzymol. 77 , 237 (1981)]. Sie sind für den Transport verschiedener Aminosäuren in Form ihrer γ-Glutamyl-Derivate, die Bildung von Polyglutaminsäure in Bacilli sowie den Abbau von Glutathion (γ-Glutamyl-Cysteinyl-Glycin) verantwortlich.

Ein Screening auf γ-Glutamyl-Transpeptidasen erbrachte viele positive Stämme, von denen einige mit Hilfe dieses Enzyms Glutaryl-7ACA spalten können (P 37 01 221.5). Ein Stamm von Bacillus subtilis "natto" war sogar in der Lage D-α-Aminoadipinyl-p-nitroanilid, einer bezüglich der Seitenkette analogen Verbindung zu CPC, zu spalten. Die Spaltung von CPC mit dieser isolierten γ-Glutamyl-Transpeptidase gelang ebenfalls (P 37 43 323.7). Aus diesen Untersuchungen folgt, daß es mindestens drei verschiedene γ-Glutamyl-Transpeptidasen gibt:

- solche, die weder Glutaryl-7ACA noch α-Aminoadipinyl-7ACA spalten, sondern nur den Glutamylrest wie z. B. in γ-Glutamyl-p-nitroanilid abspalten;
- solche, die Glutaryl-7ACA spalten und Glutamylreste abspalten;
- solche, die sowohl Glutaryl-7ACA und α-Aminodipinyl-7ACA als auch Glutamylreste abspalten.

Die Erfindung betrifft somit:

1. 7-(δ-(D-α-Aminoadipinyl)-amido)-4-methylcumarin der Formel I,

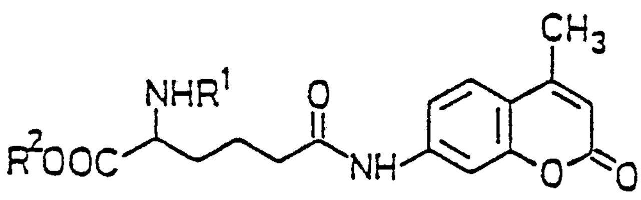

I

in der unabhängig voneinander $R^1$ Wasserstoff oder eine Acylgruppe und $R^2$ Wasserstoff oder eine Alkyl- oder Arylgruppe, wobei die Schutzgruppen jeweils substituiert sein können, bedeutet,
sowie dessen Säureadditions- oder Alkali- oder Ammoniumsalz.

2. Ein Verfahren zur Herstellung der Verbindung der Formel I, das dadurch gekennzeichnet ist, daß man mit Schutzgruppen versehene α-Aminoadipinsäure und 7-Amino-4-methylcumarin miteinander zur Reaktion bringt und gegebenenfalls die Schutzgruppen $R^1$ und $R^2$ hydrolytisch oder hydrogenolytisch abspaltet.

3. Die Verwendung der Verbindung der Formel I, in der $R^1$ und $R^2$ Wasserstoff bedeutet, sowie deren Säureadditions-oder Alkali- oder Ammoniumsalze zur Auffindung von α-Adipinylamidase Aktivität.

Im folgenden wird die Erfindung detailliert beschrieben, insbesondere in ihren bevorzugten Ausführungsformen. Ferner wird die Erfindung in den Ansprüchen definiert.

Zur Herstellung der Verbindung der Formel I, in der $R^1$ und $R^2$ Wasserstoff bedeutet, verwendet man D-α-Aminoadipinsäure und 7-Amino-4-methylcumarin als Ausgangskomponenten.

D-α-Aminoadipinsäure wird mit den Schutzgruppen $R^1$ und $R^2$, die unter milden Bedingungen leicht zu entfernen sind, versehen, und zwar nach den üblichen Methoden, wie sie beispielsweise bei J.E. Baldwin,

S.R. Hevchen, P.D. Singh, Biochem. J. 186,881 (1980) beschrieben werden.

$R^1$ und $R^2$ sind beides Schutzgruppen, die in der Peptidsynthese üblicherweise verwendet werden. Für $R^1$ können Acylgruppen, mit Alkyl- oder Arylresten, zur Verwendung kommen, die unter schwach sauren Bedingungen entfernt werden können. Bevorzugt werden tert.-Butoxycarbonylgruppen oder aromatische Gruppen der allgemeinen Formel III,

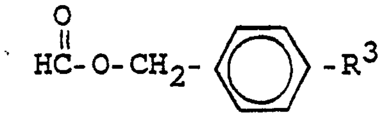

III

in der $R^3$ Nitro oder Wasserstoff sein können, eingesetzt. Diese aromatische Schutzgruppe ist durch Bromwasserstoff oder katalytische Hydrierung abzuspalten.

Die Schutzgruppe $R^2$ kann eine Alkyl- oder Arylgruppe sein, die jeweils substituiert sein kann. Vorzugsweise wird eine aromatische Schutzgruppe und zwar der allgemeinen Formel IV,

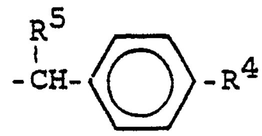

(IV)

in der unabhängig voneinander $R^4$ Nitro oder Wasserstoff und $R^5$ Phenyl oder Wasserstoff sein können, eingesetzt.

Die Kupplung der mit Schutzgruppen versehenen D-α-Aminoadipinsäure mit 7-Amino-4-methyl-cumarin wird durch üblicherweise in der Peptidchemie benutzte Verfahren erreicht:

1. Aktivierung der Carbonsäurekomponente mit Chlorameisensäureestern (C.P. Dorn, M. Zimmermann, Biochem. J. 183, 555 (1979)):

Die Kondensationsreaktion wird in der Weise durchgeführt, daß zunächst durch Zusammengeben der geschützten α-Aminoadipinsäure und Chlorameisensäureester, vorzugsweise Chlorameisensäureisobutylester, in einem organischen Lösungsmittel, vorzugsweise cyclische Ether wie Tetrahydrofuran (THF), chlorierte Kohlenwasserstoffe, wie Dichlormethan oder Chloroform, oder Amide, wie Dimethylformamid (DMF), die freie Carbonsäuregruppe bei -50 bis 20 °C, vorzugsweise -20 bis -5 °C aktiviert wird.
Durch Zugabe von äquimolaren Mengen 7-Amino-4-methyl- cumarin zu dem obigen Reaktionsgemisch wird bei einer Reaktionstemperatur von 0-40 °C, vorzugsweise 20-25 °C, die Bildung des gewünschten Reaktionsprodukts innerhalb einer Reaktionszeit von 2-10 h, vorzugsweise 3-5 h, erreicht. Das so erhaltene Reaktionsprodukt wird mit oder ohne Abdestillation des Reaktionslösungsmittels in einem mit Wasser nicht mischbaren organischen Lösungsmittel wie Chloroform, Dichlormethan, Ethylacetat, Butylacetat oder Methylisobutylketon aufgelöst. Die Lösung wird mit saurem oder alkalischem Wasser gewaschen und das Lösungsmittel zur Isolierung des Produkts entfernt. Wenn eine Reinigung erforderlich ist, dann wird aus einem geeigneten Lösungsmittel umkristallisiert oder durch Säulenchromatographie an Kieselgel oder aktivem Aluminiumoxid gereinigt.

2. Verwendung eines Kondensationsreagenzes:

Die Kupplungsreaktion wird durchgeführt in Gegenwart eines Kondensationsreagenzes wie N,N′-Dicyclohexylcarbodiimid (DCCD) (J.C. Sheekan, G.P. Hess, J. Am. Chem. Soc. 77, 1067 (1955)) oder 2-Ethoxy-1,2-dihydrochinolin-1-carbonsäureethylester (EEDQ) (B. Belleau, G. Malek, J. Am. Chem. Soc. 90, 1651 (1968)) oder vergleichbaren Reagenzien (vgl. Übersicht: Y.S. Klausner, M. Bodansky, Synthesis 1972, 453) durch Zusammengeben der Kupplungskomponenten in äquimolaren Mengen und des Reagenzes in einem organischen Lösungsmittel, vorzugsweise Amine, wie Pyridin, Amide, wie DMF, chlorierte Kohlenwasserstoffe, wie Dichlormethan oder Chloroform, oder cyclische Ether, wie THF. Der Reaktionsprozeß kann durch Kieselgel-Dünnschichtchromatographie verfolgt und nach dem Verschwinden des Ausgangsmaterials abgebrochen werden. Die Aufarbeitung des so erhaltenen Reaktionsprodukts kann wie oben beschrieben durchgeführt werden.

3. Aktivierung der Aminokomponente (S. Khammungkhune, G. Sigler, Synthesis 1980, 614):

Bevorzugte Aktivierungsmittel sind Phosphorigsäuredialkylester, insbesondere Diethylphosphit, die im Überschuß in einem inerten organischen Lösungsmittel, vorzugsweise jedoch in Diethylphosphit als Lösungsmittel, mit 7-Amino-4-methylcumarin 10-50 min. auf 80-130 °C, vorzugsweise 105-115 °C, erhitzt werden. Die Kupplung erfolgt durch Zugabe der geschützten α-Aminoadipinsäure und Erhitzen des Reaktionsgemisches auf 80-130 °C, vorzugsweise 105-115 °C, für 1-6 h. Die Isolierung des Reaktionsprodukts geschieht durch Abdestillation des Lösungsmittels und Kristallisation bzw. Reinigung durch Säulenchromatographie.

Wenn erforderlich kann nach hydrogenolytischer Abspaltung der Schutzgruppen mit Wasserstoff über einen Pd-Katalysator, das freie Aminosäureamid in ein anorganisches Salz, wie das Hydrochlorid, Sulfat oder Phosphat oder in ein organisches Salz, wie das Acetat, Propionat, Citrat oder Oxalat, oder in ein Carbonsäuresalz, wie das Natrium- oder Ammoniumsalz, übergeführt werden. Zur Reinigung der Verbindung der Formel I bedient man sich üblicher Methoden, z. B. der Umkristallisation der Salze.

Bei der Verwendung der Verbindung der Formel I als Screeningsubstrat auf γ-Glutamyl-transpeptidasen produzierende Mikroorganismen hat sich folgendes Vorgehen bewährt:

Man kultiviert z. B. Erdproben oder auch isolierte Mikroorganismen und überprüft nach dem Wachstum die Kulturlösung, die Zellen und das Kulturfiltrat, auf ihre Fähigkeiten zur Hydrolyse von 7-(δ-(D-α-Aminoadipinyl)-amido)-4-methylcumarin. Dazu inkubiert man das zu prüfende Material mit diesem Reagenz bei 25 bis 50 °C in einem pH-Bereich von 6 bis 9 in gängigen Enzympuffern, wobei Phosphat- oder Tris/HCl Puffer bevorzugt sind. Das entstandene Reaktionsprodukt, 7-Amino-4-methyl-cumarin, läßt sich nach dünnschichtchromatographischer Auftrennung des Reaktionsansatzes fluorometrisch nachweisen.

Die nachfolgenden Beispiele erläutern die Erfindung näher, ohne sie darauf zu beschränken.

Beispiel 1

a) Herstellung von 7-(δ-(α-Benzyl-N-carbobenzoxy-D-α-aminoadipinyl)-amido)-4-methylcumarin:

Zu einer Suspension von 7-Amino-4-methylcumarin (525 mg) in Diethylphosphit (3 ml) und Tributylamin (0,7 ml) wird bei 110° C eine Lösung von Phosphorpentoxid (0,97 g) in Diethylphosphit (3 ml) gegeben. Nach 20 Minuten wird α-Benzyl-N-carbobenzoxy-D-α-aminoadipinsäure (1,15 g) in Diethylphosphit (3 ml) und 85 %iger Phosphorsäure (0,34 g) zugesetzt. Nach 2 h Rühren bei 110° C wird das Reaktionsgemisch im Vakuum eingeengt, mit Wasser verrührt, filtriert und im Vakuum getrocknet. Das Rohprodukt wird in Chloroform/Methanol 1 : 1 heiß gelöst, filtriert und bis zum Beginn der Kristallisation eingeengt. Nach 15 h bei 4° C wird filtriert, mit Hexan gewaschen und getrocknet.

Ausbeute: 0,96 g (62 %),
Schmelzpunkt: 180 - 181° C (Zersetzung)
Drehwert: $[\alpha]_D^{20}$ = +14.2° (c = 1,0, DMF)
Elementaranalyse:
Ber. C 68,62, H 5,57, N 5,16
Gef. C 68,82, H 5,56, N 5,28
$C_{31}H_{30}N_2O_7$

b) Herstellung von 7-(D-α-Aminoadipinylamido)-4-methylcumarin:

Eine Lösung des nach a) hergestellten Amids (0,5 g) in 70 %iger Essigsäure wird mit Palladium auf Kohle (10 %, 0,25 g) versetzt und 4 h unter Wasserstoff gerührt. Das Reaktionsgemisch wird filtriert, eingeengt und der Rückstand aus Methanol/Wasser umkristallisiert.

Ausbeute, 0,17 g (56 %),
Schmelzpunkt: 240° C (Zersetzung)
Drehwert: $[\alpha]_D^{20}$ = - 25,2° (c = 0,1, 0,2 M $Na_2CO_3$/DMSO = 5 : 1)

Elementaranalyse: $C_{16}H_{18}8N_2O_5$
Berechnet: C 60,37 H 5,70 N 8,80
Gefunden: C 59,92 H 5,80 N 8,71

Beispiel 2

Aktivitätsbestimmungen

a) Photometrischer Standardtest für γ-Glutamyl-Transpeptidasen:

600 µl L-γ-Glutamyl-p-nitroanilid (166 µM)
300 µl Kaliumphosphatpuffer, pH 7,5, (50 mM) und
100 µl Kulturlösung werden miteinander vermischt und bei 37° C inkubiert.

$$\epsilon 405 = 9620 \times \frac{1}{\text{Mol} \times \text{cm}}$$

b) Fluorometrischer Test auf Spaltung von 7-(D-α-Aminoadipinylamido)-4-methylcumarin (AAA-AMC):

400 µl AAA-AMC (500 µM)
100 µl Probe
500 µl Kaliumphosphatpuffer, pH 7,5, (50 mM)
Anregung bei 355 nm
Emission bei 450 nm

c) DC-Test auf AAA-AMC-Spaltung

2 µl Reaktionsansatz aus b) werden auf HPTLC-platten (Kieselgel 60F254) aufgetragen.
Mobile Phase: Essigsäureethylester/Hexan (2 : 1)
Detektion :
a) UV-Lampe bei 366 nm
b) DC-Densitometer: Anregung bei 354 nm
c) Rf-Wert: 0,7

Beispiel 3

Zur Differenzierung unterschiedlicher γ-Glutamyl-Transpeptidasen (γ-GTP) wurden verschiedene Mikroorganismen (Tab. 1) in unten angeführtem Medium kultiviert und auf ihre Hydrolyseaktivitäten bezüglich γ-Glutamyl-p-nitroanilid (γ-Glu-pNA, allgemeines Substrat für γ-GTP's), γ-Glutamyl-p-nitroanilid in Gegenwart von 7-Aminocephalosporansäure (7ACA) (γ-GTP's, die Glutaryl-7ACA spalten, werden durch 7ACA gehemmt) und 7-(δ-(D-α-Aminoadipinyl)-amido)-4-methylcumarin hin überprüft. Die Hydrolyseaktivität wurde gemäß Beispiel 2 gemessen.

Tabelle 1:

| Mikroorganismus | Hydrolyse von γ-Glu-pNA | Hemmung d. Hydrolyse γ-Glu-pNA + 7ACA | Hydrolyse AAA-AMC |
|---|---|---|---|
| Proteus vulgaris ATCC 9634 | + | - | - |
| Pseudomonas fragi DSM 3881 | + | + | - |

Tabelle 1 (Forts.):

| Mikroorganismus | Hydrolyse von γ-Glu-pNA | Hemmung d. Hydrolyse γ-Glu-pNA + 7ACA | Hydrolyse AAA-AMC |
|---|---|---|---|
| Bacillus subtilis "natto" IFO 3025 | + | + | + |

Anzuchtmedium:
Pepton 1 %
Fleischextrakt 0,5 %
NaCl 0,5 %
pH 7,0
Anzuchtbedingungen: 24 Stunden; 30° C; 190 Upm

Beispiel 4

Angereicherte γ-Glutamyl-Transpeptidasen der in Beispiel 3 genannten Mikroorganismen wurden mit Desacetyl-Cephalosporin C inkubiert. Nur das Enzym aus Bacillus subtilis, welches auch AAA-AMC spaltet, kann Desacetyl-Cephalosporin C und Cephalosporin C zu D-α-Aminoadipinsäure und Desacetyl-7ACA (18 % Umsetzung) bzw. 7ACA (3 % Umsetzung) hydrolysieren.

**Ansprüche**

1. 7-(δ-(D-α-Aminoadipinyl)-amido)-4-methylcumarin der Formel I,

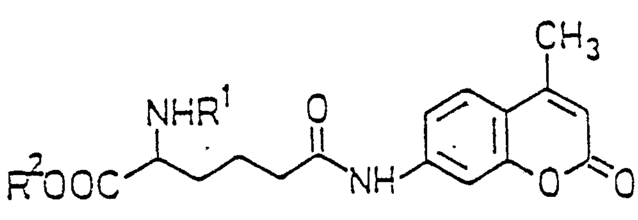

I

in der unabhängig voneinander
$R^1$ Wasserstoff oder eine Acylgruppe und
$R^2$ Wasserstoff oder eine Alkyl- oder Arylgruppe, wobei die Schutzgruppen jeweils substituiert sein können, bedeutet, sowie dessen Säureadditions- oder Alkali- oder Ammoniumsalz.

2. Verfahren zur Herstellung der Verbindung der Formel I nach Anspruch 1, dadurch gekennzeichnet, daß man mit α-Aminoadipinsäure der Formel II

$$R^2OOC-\underset{}{CH}(NHR^1)-(CH_2)_3COOH \qquad II$$

in der $R^1$ eine Acylgruppe und
$R^2$ eine Alkyl- oder Arylgruppe, wobei die Schutzgruppen jeweils substituiert sein können, bedeutet, und 7-Amino-4-methylcumarin miteinander zur Reaktion bringt und gegebenenfalls die Schutzgruppen hydrolytisch oder hydrogenolytisch abspaltet.

3. Die Verwendung der Verbindung der Formel I nach Anspruch 1, in der $R^1$ und $R^2$ Wasserstoff bedeutet, sowie deren Additions- oder Alkali- oder Ammoniumsalze zur Auffindung von α-Adipinylamidase Aktivität.

Patentanspruch für folgenden Vertragsstaat: ES

1. Verfahren zur Herstellung von
7-(γ-(D-α-Aminoadipinyl)-amido)-4-methylcumarin der Formel I,

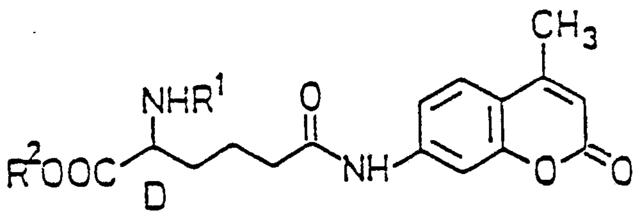

I

in der unabhängig voneinander
$R^1$ Wasserstoff oder eine Acylgruppe und
$R^2$ Wasserstoff oder eine Alkyl- oder Arylgruppe, wobei die Schutzgruppen jeweils substituiert sein können, bedeutet, sowie dessen Säureadditions- oder Alkali- oder Ammoniumsalz.
dadurch gekennzeichnet, daß man mit α-Aminoadipinsäure der Formel II

$$R^2OOC-CH(NHR^1)-(CH_2)_3COOH \qquad II$$

in der $R^1$ eine Acylgruppe und
$R^2$ eine Alkyl- oder Arylgruppe, wobei die Schutzgruppen jeweils substituiert sein können,
bedeutet,
und 7-Amino-4-methylcumarin miteinander zur Reaktion bringt und gegebenenfalls die Schutzgruppen hydrolytisch oder hydrogenolytisch abspaltet.

2. Die Verwendung der Verbindung der Formel I erhältlich nach Anspruch 1, in der $R^1$ und $R^2$ Wasserstoff bedeutet, sowie deren Additions- oder Alkali- oder Ammoniumsalze zur Auffindung von α-Adipinylamidase Aktivität.

Europäisches Patentamt

# EUROPÄISCHER RECHERCHENBERICHT

Nummer der Anmeldung

EP 89118663.7

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
| A | CHEMICAL ABSTRACTS, Band 102, Nr. 23, 10. Juni 1985, Columbus, Ohio, USA PRUSAK, E. et al. "γ-Glutamylconmarylamides and their N-acyl derivatives" Seite 634, Spalte 1, Zusammenfassung-Nr. 204 291e & Pol PL 123 466 -- | 1,2 | C 07 D 311/16<br>C 12 Q 1/48 |
| A | CHEMICAL ABSTRACTS, Band 94, Nr. 3, 19. Januar 1981, Columbus, Ohio, USA KHAMMUNGK HUNE, S. et al. "A convenient synthesis of benzyl oxycarbonyl-l-amino acid 4-methyl conmaryl-7--amides" Seite 477, Spalte 1, Zusammenfassung-Nr. 16 040q & Synthesis 1980, (8), 614-15 -- | 1,2 | RECHERCHIERTE SACHGEBIETE (Int. Cl.5) |
| A | DE - A1 - 3 614 647 (EUROPÄISCHE ATOMGEMEINSCHAFT) * Anspruch 1; Seite 4, Zeilen 16-19 * ---- | 1-3 | C 07 D 311/00<br>C 12 Q 1/00 |

Der vorliegende Recherchenbericht wurde für alle Patentansprüche erstellt.

| Recherchenort | Abschlußdatum der Recherche | Prüfer |
|---|---|---|
| WIEN | 07-12-1989 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument